# EUROPEAN PATENT APPLICATION

(11) **EP 4 632 760 A1**
(43) Date of publication of application: **15.10.2025**
(21) Application number: 23901148.9
(22) Date of filing: 08.12.2023
(51) Int. Cl.: G16H 40/40, G16B 25/00, G16B 30/10, G16B 50/00

(54) **METHOD FOR UPDATING RESEARCH AND DEVELOPMENT MODULE OF IN VITRO DIAGNOSTIC REAGENT PRODUCT AND RESEARCH AND DEVELOPMENT MANAGEMENT DEVICE**

(30) Priority: 08.12.2022 KR 20220170637
(71) Applicant: Seegene, Inc., Seoul 05548 (KR)
(72) Inventor: KIM, Youngwook, Seoul 05548 (KR); LEE, Seungheon, Seoul 05548 (KR); JEON, Chang Ha, Seoul 05548 (KR)
(74) Representative: Viering, Jentschura & Partner mbB Patent- und Rechtsanwälte
(86) International application number: PCT/KR2023/020188
(87) International publication number: WO 2024/123131

(57) **Abstract**

A method and a device for updating a research and development module are provided. The method for updating a research and development module according to one embodiment may include the steps of: obtaining research and development data of an in vitro diagnostic reagent product generated by the research and development module used by an external user account and/or clinical data of the in vitro diagnostic reagent product; obtaining update candidate content for the research and development module determined on the basis of at least a portion of the obtained research and development data and the clinical data; obtaining a result generated by performing change impact assessment of the update candidate content on the research and development module; and controlling the research and development module to be updated in response to the result generated by performing the change impact assessment.

## Description

### TECHNICAL FIELD

The present disclosure relates to a method for updating a research and development module of an in vitro diagnostic reagent product capable of updating the research and development module, and a research and development management device.

### BACKGROUND ART

In vitro diagnostics (IVD) refers to a technology that analyzes target objects such as blood, urine, or cells collected from the human body to diagnose health conditions. Such in vitro diagnostics includes immunodiagnosis, self-blood glucose measurement, molecular diagnostic technologies, etc.

Among these, the immunodiagnosis is a method that uses a specific antibody reaction to an antigen in analyzing a sample. A representative example of the immunodiagnosis is known as an enzyme-linked immunosorbent assay (ELISA). According to the ELISA, a current disease condition may be simply diagnosed. However, the ELISA is known to have slightly low sensitivity.

Molecular diagnostics is a method for diagnosing diseases by detecting and analyzing DNA or RNA which is a genetic information material. A representative example of the molecular diagnostics is known as polymerase chain reaction (PCR). In the PCR, a trace amount of specific DNA may be amplified into a large amount in a short period of time. The PCR has a higher sensitivity than the above-described immunodiagnosis. Recently, real-time polymerase chain reaction (real-time PCR) using a fluorescent probe is becoming widespread and is being used to diagnose various types of diseases.

In the in vitro diagnostics including the above-described immunodiagnosis and molecular diagnostics, reagent products for the in vitro diagnostics are classified as medical devices. Since the reagent products are the medical devices, the reagent products should obtain regulatory approval for sale in each country.

In this way, the in vitro diagnostic reagent products should be developed in various ways to diagnose various types of diseases using medical devices. However, in reality, external user accounts of various reagent products are competitively engaged in the development and production of reagent products for diagnosing well-known diseases. In addition, the reagent products developed by each external user account should use dedicated testing equipment. Therefore, multiple testing equipment should be provided in order to test the reagent products of the external user accounts.

As a result, there is a problem that it is difficult to substantially perform various tests in primary and secondary hospitals where patients may easily access. As a result, patients are unable to perform in vitro diagnostic tests at the early stage of their disease, which also leads to problems in delaying proper medical examination and treatment.

### DETAILED DESCRIPTION OF THE DISCLOSURE

### TECHNICAL PROBLEMS

The present disclosure is directed to providing a research and development management device and method for an in vitro diagnostic reagent product capable of developing customized products desired by patients with various diseases or medical institutions treating diseases.

In addition, the present disclosure is directed to providing a research and development management device and method for an in vitro diagnostic reagent product capable of easily developing a variety of in vitro diagnostic reagent products required by external user accounts in various medical departments.

In addition, the present disclosure is directed to enabling various in vitro diagnostic reagent products developed and produced by external user accounts to be tested on single testing equipment.

In addition, the present disclosure is directed to providing a research and development module in which a research and development process and a production process are implemented so that an external user account may develop and produce various in vitro diagnostic reagent products in a standardized system.

In addition, the present disclosure is directed to providing a research and development management device and method for an in vitro diagnostic reagent product capable of gradually evolving a research and development module used by an external user account by allowing the external user account to use data obtained by the same research and development process and production process.

### MEANS FOR SOLVING PROBLEMS

According to a method for updating a research and development module may include: obtaining, by the research and development management device, research and development data and/or clinical data of the in vitro diagnostic reagent product generated by the research and development module used by an external user account; obtaining update candidate content for updating the research and development module determined at least partially based on the obtained research and development data and clinical data; obtaining a result of performing change impact assessment of the update candidate content on the research and development module; and controlling the research and development module to be updated in response to the result of performing the change impact assessment.

In one embodiment, when the result of performing the change impact assessment satisfies a predetermined criterion, the controlling of the research and development module to be updated may be performed to updeate the research and development module.

In one embodiment, the research and development module may include software with an implemented algorithm for designing an oligonucleotide used in a target nucleic acid amplification reaction.

In one embodiment, the update candidate content may include at least one selected from the group consisting of an algorithm for determining an oligonucleotide designable region of a target sequence, an algorithm for designing the oligonucleotide, and an algorithm for calculating an assessment score for performance of the oligonucleotide.

In one embodiment, the research and development module may include software with an implemented algorithm used to process amplification data obtained from the target nucleic acid amplification reaction.

In one embodiment, the update candidate content may include at least one selected from the group consisting of a baselining algorithm, an amplification curve fitting algorithm, an algorithm for determining a Ct value, a melting curve analysis algorithm.

In one embodiment, the research and development module includes software implementing an algorithm used to determine a presence or absence of a target analyte within a sample.

In one embodiment, the update candidate content may include at least one of an algorithm for determining presence or absence of a target analyte using a Ct value, and an algorithm for determining the presence or absence of the target analyte using an amplification curve fitting result.

In one embodiment, the research and development data and/or the clinical data of the in vitro diagnostic reagent product may include at least one selected form the group consisting of target sequence data, an experimental reaction results for combinations of the oligonucleotides, amplification data obtained from a nucleic acid amplification reaction, trouble data generated during research and development of the reagent product, an instruction executed to operate research and development equipment, a value of a parameter referenced in the execution of the instructions, and trouble shooting data corresponding to the trouble data

In one embodiment, the research and development module may be software used for operating research and development equipment.

In one embodiment, the research and development module may be at least one of a nucleic acid extraction device, a liquid handler, and a real-time PCR (polymerase chain reaction) device.

In one embodiment, an external user account may be provided with the same research and development process, allowing the research and development of the reagent product to be carried out using the same technology, and may use research and development equipment for performing the research and development of the reagent product in accordance with the same research and development process

In one embodiment, the same technology includes at least one technology selected from the group consisting of the following technologies: a technology for determining a target sequence and an amplicon; a candidate nucleotide design technology for primers and probes; an oligonucleotide structure technology; a signal generation mechanism indicating the presence of a target; a technology for differentiating multiple signals generated from a single label in a single channel; signal processing technology and positive/negative determination technology; and nucleic acid extraction technology.

In one embodiment, the same research and development process may be a standardized research and development process provided by the research and development management device of the in vitro diagnostic reagent product.

In one embodiment, the research and development equipment may include at least one selected from the group consisting of a nucleic acid extraction device, a liquid handler, and a real-time polymerase chain reaction (PCR) device.

In one embodiment, the research and development equipment may be the same equipment, and the same equipment may be calibrated with the same calibration

In one embodiment, the research and development module is provided in the research and development management device so that the external user account accesses and uses the research and development module, or is distributed from the research and development management device to a terminal of the external user account.

In one embodiment, the research and development data and/or the clinical data of the in vitro diagnostic reagent product may be obtained from a research and development support module included in the research and development management device and/or a research and development support device connected to the research and development management device via a communication network.

A research and development management device according to another embodiment may include a memory that stores at least one instruction; and a processor, wherein, by executing the at least one instruction, the processor controls a research and development management device to obtain research and development data of an in vitro diagnostic reagent product and/or clinical data of the in vitro diagnostic reagent product that is generated by a research and development module used by an external user account, obtain update candidate content for updating the research and development module determined at least partially based on the obtained research and development data and clinical data, obtain a result of performing change impact assessment of the update candidate content on the research and development module, and update the research and development module in response to the result of performing the change impact assessment.

A computer-readable recording medium storing a computer program according to further another embodiment, the computer program is programmed to perform the following steps: obtaining, by a research and development management device, research and development data and/or clinical data of the in vitro diagnostic reagent product generated by a research and development module used by an external user account; obtaining update candidate content for updating the research and development module determined at least partially based on the obtained research and development data and clinical data; obtaining a result of performing change impact assessment of the update candidate content on the research and development module; and controlling the research and development module to be updated in response to the result of performing the change impact assessment.

A computer program stored in a computer-readable recording medium according to still further another embodiment, the computer program is programmed to perform the following steps: obtaining, by a research and development management device, research and development data and/or clinical data of the in vitro diagnostic reagent product generated by a research and development module used by an external user account; obtaining update candidate content for updating the research and development module determined at least partially based on the obtained research and development data and clinical data; obtaining a result of performing change impact assessment of the update candidate content on the research and development module; and controlling the research and development module to be updated in response to the result of performing the change impact assessment.

### EFFECTS OF INVENTION

According to one embodiment of the present disclosure, an external user account participating in reagent development through a research and development management device of an in vitro diagnostic reagent product can use a gradually evolved research and development module.

According to another embodiment of the present disclosure, it is possible to obtain various data, which can evolve a research and development module, from an external user account.

According to still another embodiment of the present disclosure, since external user accounts may perform research and development using the evolved research and development module, it is possible to facilitate research and development of an improved in vitro diagnostic reagent product.

### BRIEF DESCRIPTION OF THE DRAWINGS

FIG. 1 is a diagram conceptually illustrating a relationship between a research and development management device and an external user account using the research and development module according to one embodiment.
FIG. 2 is a block diagram illustrating components included in the research and development management device according to one embodiment.
FIG. 3 is a diagram for describing an operation of obtaining research and development data and clinical data generated while using the research and development module from the external user account according to one embodiment.
FIG. 4 is a diagram for describing an operation of controlling update candidate content to perform an update in response to a change impact assessment according to one embodiment.
FIG. 5 is a diagram for describing an example of update candidate content according to one embodiment.
FIG. 6 is a diagram for describing an example of a result of performing a change impact assessment according to one embodiment.
FIG. 7 is a diagram for describing an operation of a research and development management device using a development support device that supports an operation of a research and development module according to another embodiment.
FIG. 8 is a diagram illustrating a method for updating a research and development module according to one embodiment.
FIG. 9 is a flowchart for describing in more detail some steps of FIG. 8.

### BEST MODE FOR IMPLEMENTING THE INVENTION

Advantages and features of the present invention, and methods for achieving them, will become clear with reference to the embodiments described below in detail in conjunction with the accompanying drawings. However, the present invention is not limited to the embodiments disclosed below, but may be implemented in various different forms, and only the present embodiments make the disclosure of the present invention complete, and common knowledge in the art to which the present invention belongs It is provided to fully inform the holder of the scope of the invention, and the present invention is only defined by the scope of the claims.

In the description of the present invention, if it is determined that a detailed description of a related known function or configuration may unnecessarily obscure the subject matter of the present invention, the detailed description will be omitted. In addition, terms to be described later are terms defined in consideration of functions in the present invention, which may vary according to the intention or custom of a user or operator. Therefore, the definition should be made based on the contents throughout this specification.

The term 'external user account' used herein includes a company that researches and develops an in vitro diagnostic reagent product through a research and development management device or produces an in vitro diagnostic reagent whose research and development has been completed as a product.

In one embodiment of the present disclosure, the 'external user account' may include a company that performs the research and development and production of the in vitro diagnostic reagent product.

The term 'in vitro diagnostic reagent product' used herein includes a molecular diagnostic reagent product.

The term 'in vitro diagnostic reagent' used herein includes a molecular diagnostic reagent or a reagent for detecting target nucleic acids.

The term 'in vitro diagnostic' used herein refers to a technology that rapidly diagnoses diseases outside the body using substances derived from the human body such as blood, excretions, body fluids, and saliva. The in vitro diagnostics play an important role in clinical decision-making and are becoming an essential and specialized element in patient treatment. Representative examples of the in vitro diagnostics may include blood sugar measurements, pregnancy tests, and COVID-19 tests, and equipment for performing the in vitro diagnostics is called in a vitro diagnostics (IVD) device. The in vitro diagnostics devices are medical devices used for testing using specimens such as tissue, blood, and urine collected from the human body for the purpose of diagnosing and prognosing diseases, assessing health conditions, determining the effectiveness of disease treatment, and preventing diseases. The in vitro diagnostics devices also include in vitro diagnostic reagent products.

In the vitro diagnostics devices may be classified as follows according to the testing method:
i) Immunochemistry
ii) Self-monitoring Blood Glucose (SMBG)
iii) Point of Care Testing (POCT)
iv) Molecular Diagnostics
v) Hematology
vi) Clinical Chemistry
vii) Hemostasis or Coagulation
viii) Tissue Diagnostics

In addition, the in vitro diagnostic reagent products may be multiplex molecular diagnostic reagent products for detecting multiple targets.

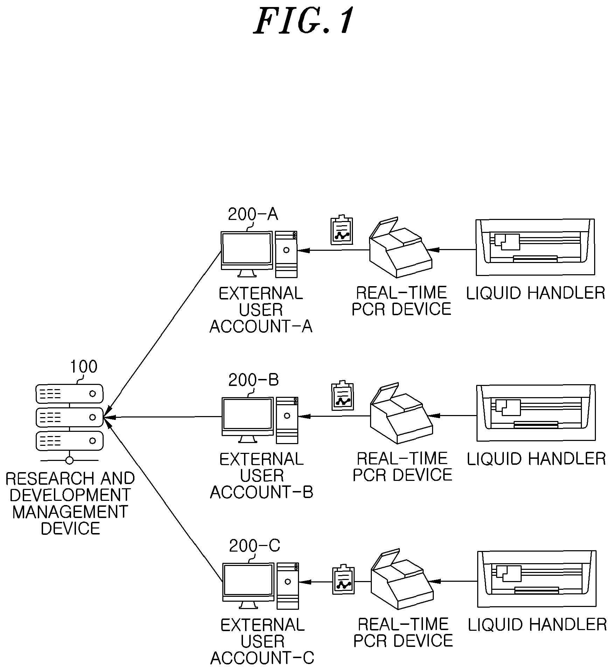

FIG. 1 is a diagram conceptually illustrating a relationship between a research and development management device 100 and an external user account 200 using the research and development module according to one embodiment.

Here, FIG. 1 is merely an example, and the idea of the present disclosure is not construed as being limited to what is illustrated in FIG. 1. For example, the number of external user accounts 200 connected to the research and development management device 100 may be 2 or less or 4 or more. Hereinafter, FIG. 1 will be described in more detail.

Referring to FIG. 1, the research and development management device 100 and the external user account 200 may be connected to each other through a network (not shown) and communicate with each other. Here, the network includes a wireless or wired network. Among these, examples of the wireless network may include at least one of long-term evolution (LTE), LTE advance (LTE-A), code division multiple access (CDMA), wideband CDMA (WCDMA), universal mobile telecommunications system (UMTS), wireless broadband (WiBro), wireless fidelity (WiFi), Bluetooth, near field communication (NFC), or global navigation satellite system (GNSS), etc. In addition, examples of the wired network may include at least one of a universal serial bus (USB), a high definition multimedia interface (HDMI), recommended standard 232 (RS-232), a local area network (LAN), a wide area network (WAN), Internet, a telephone network, etc.

The research and development management device 100 may transmit and receive information to and from the external user account 200 and control to update the research and development module used by the external user account 200. In one embodiment, the external user account 200 may be provided with the same research and development process that allows the research and development of the reagent product to be performed using the same technology, and use research and development equipment for research and development of the reagent product according to the same research and development process, which will be described in detail later.

The external user account 200 is an account for researching and developing the in vitro diagnostic reagent product. The external user account 200 is an account that may access and log in to the research and development management device 100. The external user account 200 is an account used by using a terminal, a server, a smartphone, a desktop, a laptop, a tablet, a wearable device, etc., that may communicate with the research and development management device 100, but is not limited thereto. In one embodiment, the external user account 200 may transmit and receive information to and from the research and development management device 100 by using external research and development equipment or an external research and development devices.

The research and development management device 100 may obtain research and development data of the in vitro diagnostic reagent product and/or clinical data of the in vitro diagnostic reagent product that is generated by the research and development module.

In this specification, the research and development module is a module used in the research and development process of the reagent product of the external user account 200. The research and development module may generate the research and development data of the in vitro diagnostic reagent product and/or the clinical data of the in vitro diagnostic reagent product.

The research and development data and/or clinical data may be data of the in vitro diagnostic reagent product generated by the research and development module used by the external user account 200.

Specifically, the research and development data may refer to various data that may be generated during the research and development process of the in vitro diagnostic reagent product of the research and development module used by the external user account 200.

The clinical data may refer to various data that are generated during the clinical process of the in vitro diagnostic reagent product of the external user account 200. In one embodiment, the research and development data and clinical data may be distinguished based on whether the research and development data and clinical data are generated during the research and development phase or the clinical phase, but are not limited thereto. Detailed examples of the research and development module, and the research and development data and clinical data that are generated by the research and development module will be described later.

The research and development management device 100 may obtain update candidate content determined at least partially based on the obtained research and development data and clinical data. The update candidate content is content to be used for updating the research and development module, and means an object that has been preliminarily updated.

The update candidate content may include modifications, creations, or deletions of at least one selected from the group consisting of source code, parameters, parameter values, reference values for determining the presence or absence of target analytes, and screen interfaces, all of which are included in the research and development module.

The update candidate content may be software included in the research and development module, a process included in the research and development module, a protocol included in the research and development module, a patch file applied to the research and development module, etc., but is not limited thereto. Detailed examples of the update candidate content will be described later.

The research and development management device 100 may obtain the results of performing a change impact assessment of the update candidate content on the research and development module of the update candidate content, and control the research and development module to be updated in response to the results of performing the change impact assessment.

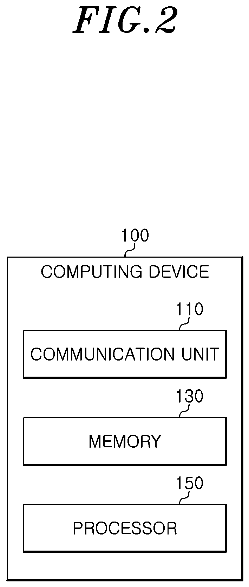

FIG. 2 is a block diagram illustrating components included in the research and development management device 100 according to one embodiment.

The research and development management device 100 may include a communication unit 110, a memory 130, and a processor 150. The research and development management device 100 may be implemented in a computing device. Hereinafter, each component will be described.

The communication unit 110 is a means for the research and development management device 100 to communicate with an external entity such as the external user account 200. The communication unit 110 can be configured as a wired module or a wireless module.

The memory 130 is implemented to include a storage medium that stores data or commands. Examples of the storage medium may include a RAM, a ROM, or a flash memory 130. Data or at least one command stored in the memory 130 may be utilized by the processor 150 described below. The memory 130 may store research and development data, clinical data, update candidate content, and commands for controlling to update the research and development module.

The processor 150 is implemented to include a calculation module that operates to perform functions to be described below using the data or commands stored in the memory 130. Examples of the calculation module may include a CPU, a GPU, etc., and may be implemented to include at least one core. Hereinafter, functions that can be performed by the processor 150 will be described. However, the functions to be described below are merely an example.

The processor 150 may control to obtain the research and development data and/or clinical data of the in vitro diagnostic reagent product. The processor 150 may control to obtain the update candidate content for the research and development module determined at least partially based on the obtained research and development data and clinical data, and control to obtain the results of performing the change impact assessment on the research and development module. In addition, the processor 150 may control the research and development module to be updated in response to the results of performing the change impact assessment.

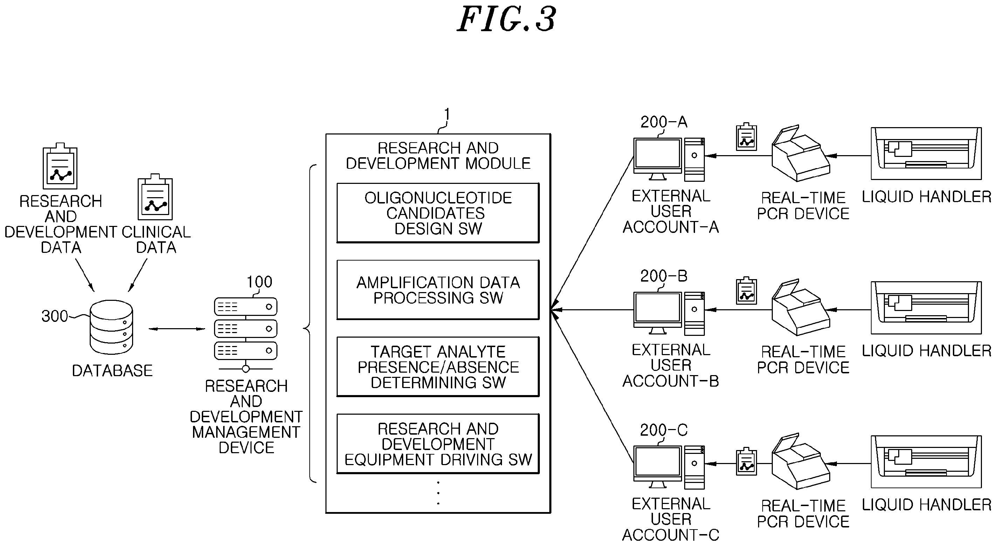

The operation of the research and development management device 100 has been briefly described so far. Hereinafter, the operation of the research and development management device 100 will be described in detail with reference to FIGS. 3 to 5.

FIG. 3 is a diagram for describing an operation of obtaining the research and development data and clinical data generated while using a research and development module 1 from the external user account 200 according to one embodiment.

In one embodiment, the use of the research and development module 1 by the external user account 200 may mean that the external user account 200 is provided with the same research and development process that enables the research and development of reagent products to be carried out using the same technology, and that the external user uses research and development equipment in accordance with the same research and development process for the development of the reagent products.

Here, the same technology for the research and development of the reagent product may be at least one of a technology for determining a target sequence and an oligonucleotide designable region, an oligonucleotide design technology for primers and probes, an oligonucleotide structure, a signal generation mechanism indicating presence of a target, a technology for differentiating multiple signals generated from one label in one channel, a signal processing technology, a positive/negative determination technology, or a nucleic acid extraction technology.

Here, the same research and development process may be a standardized research and development process provided by the research and development management device 100 of the in vitro diagnostic reagent product.

Here, the research and development equipment may be at least one of a nucleic acid extraction device, a liquid handler, or a real-time polymerase chain reaction (PCR) device. In one embodiment, the research and development equipment may be the same equipment, and the same equipment may be equipment to which the same calibration is applied. The same calibration may be measured by the same calibration tools, and the calibration may be performed by the same standard value.

For example, the same research and development equipment may be measured using the same calibration tools, and when the measured result value satisfies an error range presented by the same standard value, the measured research and development equipment may be continuously used. In addition, when the measured result value is out of the error range, the calibration by the same standard value may be performed, and the calibrated research and development equipment may be used.

The aforementioned calibration-related matters are applicable when the research and development equipment is the same equipment and is used under the same calibration standards.

In another implementation example, the research and development equipment is the same equipment, and the same equipment may operate by the same driving software. The driving software may be installed in the research and development equipment or a control terminal of the research and development equipment to operate a driving system that constitutes the research and development equipment. Therefore, the same driving software may control each of the pieces of same research and development equipment to perform uniformly according to the same control commands so that the performance of the driving system may be uniformly achieved.

The research and development data obtained from each external user account 200 may be generated from the same driving software and/or the same analysis software for each of the same type of pieces of research and development equipment described above. Accordingly, the research and development data of each external user account 200 may have data compatibility so that the research and development data may be compared with each other.

In another implementation example, the same type of pieces of research and development equipment may be released with the same manufacturer and model name or may be equivalently updated research and development equipment.

For example, in the implementation example described above, when the research and development equipment has two liquid handlers in one external user account 200, each liquid handler may be defined as the same type of pieces of research and development equipment. In addition, when two or more external user accounts 200 each have one or more liquid handlers, each liquid handler may be defined as the same type of pieces of research and development equipment.

The research and development equipment should be calibrated using the same standard value, i.e., standardized calibration data, to provide uniform and continuous performance. In particular, medical devices, etc., should be periodically calibrated according to the National Standards Basic Act. For the calibration of such research and development equipment, standardized calibration data may be used according to the same standard value.

The research and development equipment may achieve the same performance and thus produce the same research and development results only when they are calibrated with the calibration data of the same standard value and operated by the same driving software. Therefore, each of the pieces of same research and development equipment should be operated by the same driving software and use the same driving software to which the same version of update is applied.

In one embodiment, the research and development module 1 may refer to software related to research and development or clinical trials of the in vitro diagnostic reagent product.

The research and development module 1 may be software with an implemented algorithm for designing oligonucleotide used in a target nucleic acid amplification reaction. The oligonucleotide design software may be the software with the implemented algorithm for designing oligonucleotide used in the target nucleic acid amplification reaction.

In one embodiment, the algorithm for designing the oligonucleotide may include at least one of an algorithm for determining a region of a target sequence, an algorithm for designing oligonucleotide, or an algorithm for calculating an assessment score for performance of oligonucleotides, but is not limited thereto.

The oligonucleotide design software may include an algorithm to which at least one selected from the group consisting of a target sequence and amplicon determination technology capable of designing multiple oligonucleotides and an oligonucleotide candidates design technology for primers and/or probes are applied.

The research and development module 1 may be amplification data processing software. The amplification data processing software may be software with an implemented algorithm used to process the amplification data obtained from the target nucleic acid amplification reaction.

In one embodiment, the algorithm used to process the amplification data obtained from the target nucleic acid amplification reaction may be at least one of a baselining algorithm, an amplification curve fitting algorithm, a Ct value determination algorithm, or a melting curve analysis algorithm, but is not limited thereto.

In the real-time PCR reaction, the baseline refers to a signal in an initial cycle of the PCR reaction. Typically, the baseline ranges from about 3 to 15 cycles. In this case, a change in a fluorescence signal does not appear significantly. The low signal of the baseline may be the background or noise of the amplification reaction. In the real-time PCR, the baseline may be set by automatically analyzing the user's analysis or amplification curve. Since the baseline setting affects the determination of an accurate Ct value, the baseline should be set carefully. In this case, a baselining algorithm automatically analyzes the amplification curve to set the baseline.

The amplification curve fitting algorithm described above is also called 'fitted curve' or 'curve fitting' and may produce a curve that best matches data points of the amplification data set. According to one embodiment of the present disclosure, the amplification curve fitting algorithm may be expressed as a mathematical function. According to one embodiment of the present disclosure, the amplification curve fitting algorithm may be performed using a mathematical function selected from the group consisting of a polynomial function, an exponential function, a logarithmic function, a sigmoid function, a logistic function, and a Gompertz function.

The cycle threshold (Ct) value means a cycle number of a fluorescence signal indicating a contact point with the threshold. The cycle threshold refers to a value used to measure the amount of template. Theoretically, doubling the amount of template causes the Ct value to appear one cycle earlier. The above-described baseline is a weak signal that occurs at the beginning of the PCR, and the point at which the pattern of increase in the amplification signal is clearly distinguished is called the threshold. In this case, the Ct value determination algorithm is to obtain the Ct value, which is the cycle number that indicates the contact point with the threshold, and may be implemented in the analysis software.

Melting analysis means obtaining a signal indicating the presence of an extended duplex through melting of the extended duplex, and includes a method for measuring signals at two different temperatures, melting curve analysis, and melting pattern analysis. In the present disclosure, the algorithm for the melting curve analysis described above is included in the analysis software, but the algorithm for the melting pattern analysis may also be included in the analysis software, if necessary.

The melting curve or a hybridization curve may be obtained by the conventional techniques, such as those described in, for example, U.S. Patent Nos. 6,174,670 and 5,789,167, Drobyshev et al., Gene 188:45 (1997); Kochinsky and Mirzabekov, Human Mutation 19:343 (2002); Livehits et al., J. Biomol. Structure Dynam. 11:783 (1994); and Howell et al., Nature Biotechnology 17:87 (1999).

The positive/negative determination algorithm may process data obtained from the amplification reaction and perform the positive/negative determination of the target based on the Ct value and the pattern of increase in the fluorescence signal. A positive control substance used for the positive/negative determination should show positive results, and a negative control substance should show negative results. The positive/negative determination algorithm determines that the target is positive when the Ct value of the target is less than or equal to the determined Ct value. However, when the results of the positive and negative control substances are different from the expected results, the results may be determined as invalid regardless of the target results.

The research and development module 1 may be software for determining the presence or absence of a target analyte, or software for operating research and development equipment. The software for determining the presence or absence of the target analyte may be software in which an algorithm used to detect the presence or absence of the target analyte in a sample is implemented.

In one embodiment, the algorithm used for determining the presence or absence of the target analytes in the sample may be at least one of the algorithm determining the presence or absence of the target analytes using the Ct value and the algorithm for determining the presence or absence of the target analytes using fitting of amplification data obtained from the target nucleic acid amplification reaction, but is not limited thereto.

In one embodiment, the research and development equipment driving software may be software for driving at least one of a nucleic acid extraction device, a liquid handler, or a real-time polymerase chain reaction (PCR) device.

In one embodiment, the research and development modules 1 described above may be provided in the research and development management device 100 or distributed to a terminal of the external user account 200. For example, the research and development module 1 may be provided in the research and development management device 100 so that the external user account 200 may access and use the research and development module 1, or software that is distributed from the research and development management device 100 to the terminal of the external user account 200.

Specifically, the research and development module 1 may be provided in the research and development management device 100, and the external user account 200 may access the research and development management device 100 and then use the research and development module 1. In one embodiment, the research and development module 1 may be implemented as a web-app or web service that may be used after the development equipment accesses the research and development management device 100 via the Internet, but is not limited thereto. When the research and development module 1 is provided in the research and development management device 100, the research and development data and/or clinical data generated during the process in which the external user account 200 accesses the research and development management device 100 and uses the research and development module 1 may be stored in the research and development management device 100.

The research and development module 1 may be provided in the research and development management device 100. In one embodiment, when the research and development module 1 is the oligonucleotide design software or the amplification data processing software, the research and development module 1 may be provided in the research and development management device 100.

Alternatively, the research and development module 1 may be distributed to the terminal of the external user account 200 and operated by the external user account 200. In this case, the research and development module 1 may be implemented in the form of the distribution software that may be installed, executed, and operated on the terminal of the external user account 200, but is not limited thereto. When the research and development module 1 is distributed to the terminal of the external user account 200 and operated on the terminal of the external user account 200, the research and development data and/or clinical data, which is generated by the external user account 200, may be transmitted from the research and development module 1 to the research and development management device 100.

The research and development data and/or clinical data may be target sequence data, performance test results for oligonucleotides, amplification data obtained from a nucleic acid amplification reaction, trouble data generated during the research and development of a reagent product, instructions executed to operate the research and development equipment, values of parameters referenced in the execution of the instructions, and trouble shooting data corresponding to the trouble data.

The research and development data and/or clinical data may be at least one of amplification curve slope data, Ct value data, end relative fluorescence unit (RFU) data, end RFU reading data, amplification efficiency data, exclusivity and inclusivity data of sensitivity and specificity, or setting values and positive/negative determination data of analysis software.

The research and development data and/or clinical data may include a traceable data log. The data log includes at least one piece of information selected from the group consisting of date, time, generated device, user, and change contents corresponding to the generation, viewing, output, modification, and/or deletion of the research and development data.

In one embodiment, when the research and development module 1 is software for processing amplification data, software for determining the presence or absence of a target analyte, or software for operating research and development equipment, the research and development module 1 may be distributed to the terminal of the external user account 200 and operated on the terminal of the external user account 200.

As described above, the external user account 200 may be an account that directly operates and manages at least one of the nucleic acid extraction device, the liquid handler, or the real-time polymerase chain reaction (PCR) device. The external user account 200 may also be an account that also operates the driving software capable of driving the nucleic acid extraction device, the liquid handler, and the real-time PCR device. That is, the external user account 200 may operate at least one of the driving software capable of driving the nucleic acid extraction device and the liquid handler, software for processing the amplification data obtained from the real-time PCR device, and software for determining the presence or absence of the target analyte.

The research and development management device 100 may obtain the research and development data and/or clinical data, and the method for obtaining data may differ depending on the type of the research and development module 1. The research and development management device 100 may store, in a database, the research and development data and/or clinical data generated from the research and development module 1 that is directly operated by the research and development management device 100. For example, when the research and development module 1 is the oligonucleotide design software or the amplification data processing software, the research and development management device 100 may obtain the target sequence data, the experimental reaction results for a combination of the oligonucleotides, and the amplification data obtained from the nucleic acid amplification reaction. In this case, the research and development management device 100 may directly obtain these data because it directly operates the research and development module 1.

The research and development management device 100 may receive the research and development data and/or clinical data generated from the research and development module 1 distributed to the external user account 200 and store the received research and development data and/or clinical data in the database. For example, when the research and development module 1 is the amplification data processing software, the target analyte presence/absence determination software, or the research and development equipment driving software that is distributed to the external user account 200, at least one of the amplification data obtained from the nucleic acid amplification reaction, the trouble data generated during the research and development of the reagent product, the instructions executed to operate the research and development equipment and the values of the parameters referenced in the execution of the instructions, or the trouble shooting data corresponding to the trouble data may be received from the external user account 200. In one embodiment, the research and development management device 100 may obtain the data from the terminal of the external user account 200, or obtain the data generated by the external user account from the research and development management device 100 itself.

The external user account 200 may discover various problems in the research and development process while performing the research and development for developing the in vitro diagnostic reagent. In this case, the trouble data, which is a problem occurring by the external user account 200, may be generated. The research and development management device 100 may obtain the trouble data and store the obtained trouble data in the database 300.

The trouble shooting data corresponding to the trouble data described above may be generated from the external user account 200 that generates the trouble data. That is, any one of the external user accounts 200 that generate the trouble data may generate the trouble shooting data by resolving the problem of the corresponding trouble data. Therefore, any one of the external user accounts 200 described above may generate the trouble shooting data corresponding to the trouble data, respectively.

In one embodiment, the trouble shooting data corresponding to the trouble data described above may be generated from other external user accounts 200. That is, when the trouble data is generated from any one of the external user accounts 200, the corresponding trouble data may be provided to other external user accounts 200. One or more of the external user accounts 200 among the other external user accounts 200 may resolve the problem of the corresponding trouble data, thereby generating the trouble shooting data. Therefore, any one of the external user accounts 200 described above may generate the trouble data, and other external user accounts 200 may generate the trouble shooting data corresponding to the trouble data described above.

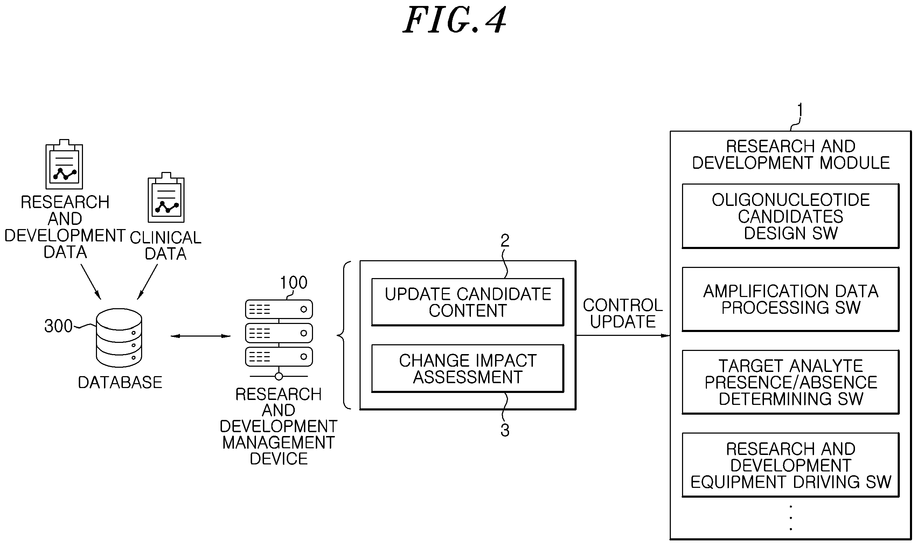

So far, the operation of the research and development management device 100 obtaining the data generated using the research and development module 1 has been described with reference to FIG. 3. Hereinafter, the operation of controlling the research and development management device 100 to determine the update candidate content 2 and update the research and development module 1 according to the change impact assessment 3 will be described with reference to FIGS. 4 to 6.

In FIG. 4, the research and development management device 100 may determine the update candidate content 2 using the obtained research and development data and/or clinical data. The research and development management device 100 may obtain the results of performing the change impact assessment 3 and control the research and development module 1 to be updated in response to the change impact assessment.

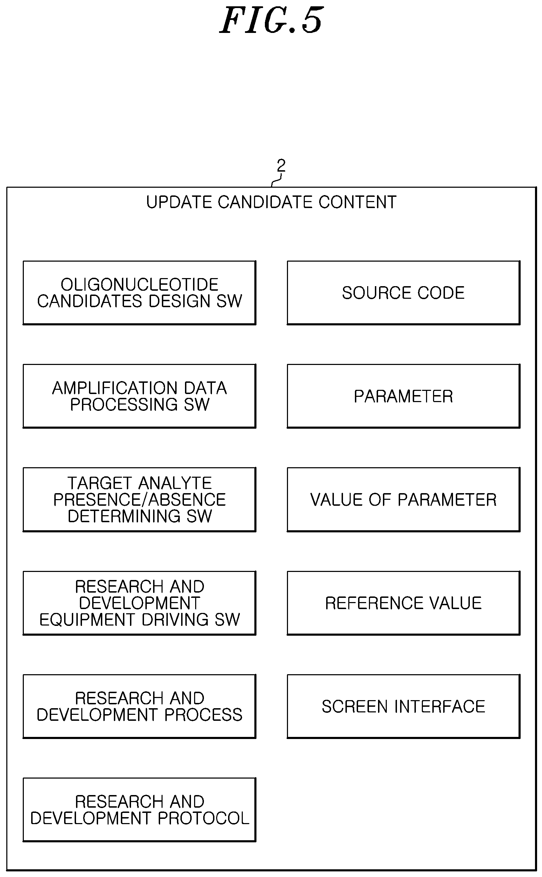

Here, the update candidate content 2 refers to content to be preliminarily updated, and the update candidate content 2 will be described in detail with reference to FIG. 5.

The update candidate content 2 may include modifications, creations, or deletions of at least one selected from the group consisting of source codes, parameters, values of parameters, reference values for determining the presence or absence of target analytes, or screen interfaces, which are included in the research and development module.

The update candidate content 2 may be update content for improving the results of detecting at least one of the multiple targets.

The update candidate content 2 may be software, an algorithm, a process, a protocol, etc. The update candidate content 2 may be at least one of software, an algorithm, a research and development process, or a research and development protocol.

The update candidate content 2 may be preliminarily updated software, a preliminarily updated algorithm, a preliminarily determined process, or a preliminarily determined research and development protocol. The update candidate content 2 may be the updated research and development module 1 that may replace the existing research and development module 1, an updated configuration that may replace a configuration included in the research and development module 1, or the patch file that may update the research and development module 1.

The update candidate content 2 may be at least one of the oligonucleotide design software, the amplification data processing software, the target analyte presence/absence determination software, or the research and development equipment driving software.

The update candidate content 2 may be the algorithm for designing the oligonucleotide used in the target nucleic acid amplification reaction included in the oligonucleotide design software. In one embodiment, the algorithm may be the algorithm for determining the region of the target sequence, the algorithm for designing the oligonucleotide, or the algorithm for calculating the assessment score for the performance of the oligonucleotides.

The update candidate content 2 may be the algorithm used to process the amplification data obtained from the target nucleic acid amplification reaction included in the amplification data processing software. In one embodiment, the algorithm may be the baselining algorithm, the amplification curve fitting algorithm, the Ct value determination algorithm, or the melting curve analysis algorithm.

The update candidate content 2 may be the algorithm for reading the presence/absence of the target analyte in the sample included in the target analyte presence/absence reading software. In one embodiment, the update candidate content 2 may be the algorithm for determining the presence or absence of the target analytes using the Ct value or the algorithm for determining the presence or absence of the target analytes using the fitting of the amplification data obtained from the target nucleic acid amplification reaction.

The update candidate content 2 may include the research and development process of the in vitro diagnostic reagent product. For example, the update candidate content 2 may include, but is not limited to, research and development order information of the in vitro diagnostic reagent product, step information, time information for each step, research and development procedure information, regulatory approval procedure information, consumable order procedure information, algorithm information for each reagent product, target information, algorithm information for each target, etc.

The update candidate content 2 may include the research and development protocol of the in vitro diagnostic reagent product. For example, the update candidate content 2 may include, but is not limited to, consumable information, temperature information, regulatory approval regulation information, equipment model information, enzyme information, buffer information, and mixing ratio information required in the research and development process of the in vitro diagnostic reagent product.

The update candidate content 2 may include at least one of source code-related syntaxes, such as conditional statements, operations, nodes, edges connecting the nodes, cycles, or regressions included in the algorithm.

The update candidate content 2 may include, but is not limited to, data weights, data structures, database schemes, parameter types, and values of parameters.

For example, the update candidate content 2 may be a modification of the value of a parameter used in the algorithm for determining the presence or absence of a target analyte.

For example, the update candidate content 2 may be a change in an instruction for changing an operation of extraction equipment in a process of extracting nucleic acids. Alternatively, the update candidate content 2 may be a change in consumable information for changing consumables in a process of preparing a sample. In addition, the update candidate content 2 may be information on a newly added reagent product in the software for determining the presence or absence of a target analyte.

Referring back to FIG. 4, the research and development management device 100 may determine the update candidate content 2 based on the research and development data and clinical data. For example, the research and development management device 100 may determine the update candidate content 2 used to update the research and development module 1 based on the research and development data and clinical data that are generated by the research and development module 1. The update candidate content 2 may be software or algorithm with improved performance, cost, speed, etc. compared to the existing software or algorithm, or may be an efficient process or protocol, code, or screen interface compared to the existing process or protocol, code, or screen interface, but is not limited thereto.

The research and development management device 100 may obtain the results of performing the change impact assessment 3 of the update candidate content 2 on the research and development module 1 of the update candidate content 2.

The change impact assessment 3 may be the impact assessment of the results of analyzing the performance of the in vitro diagnostic reagent product using the research and development module.

The change impact assessment 3 may assess whether, in the results of detecting the molecular diagnostic reagent product using the research and development module to which the update candidate content has been applied, the detection results for the remaining targets, excluding at least one target with improved detection results, are equivalent.

The change impact assessment 3 refers to information that analyzes the potential impact on a series of research and development processes, regulatory approval, performance, etc., involved in the research and development of an in vitro diagnostic reagent product, when the update candidate content 2 is reflected in the research and development module 1. The results of performing the change impact assessment 3 are information that includes the information generated while performing the change impact assessment 3.

The change impact assessment 3 may be performed using at least one selected from the group consisting of a Ct value, an End-RFU value, sensitivity, and positive/negative determination results from use in the molecular diagnostic reagent product.

For example, the results of performing the change impact assessment 3 may include letters, numbers, symbols, graphs, amplification curves, numerical comparison tables, geometric figures, etc., that indicate the impact on the research and development module 1 or the reagent product, when the research and development module 1 is updated using the update candidate content 2. In one embodiment, the results of performing the change impact assessment 3 may be information that includes changed contents, changed values, changed parameters, values of the changed parameters, exclusivity and inclusivity data of sensitivity and specificity, setting values and positive/negative determination data of the analysis software, positive/negative determination criteria, analysis accuracy, analysis speed, an opinion of a person in charge on the impact of the change on the research and development module 1, etc., but is not limited thereto.

In another embodiment, the results of performing the change impact assessment 3 may be a value indicating whether to update the research and development module 1 as 'yes' or 'no'. For example, when the change impact assessment 3 is information that allows performing the update of the research and development module 1 using the update candidate content 2, the results of performing the change impact assessment 3 may include the information 'yes', and when the change impact assessment 3 is information that denies performing the update of the research and development module 1 using the update candidate content 2, the results of performing the change impact assessment 3 may include the information 'no'. Alternatively, the results of performing the change impact assessment 3 may include binary information such as 1 or 0, but is not limited thereto.

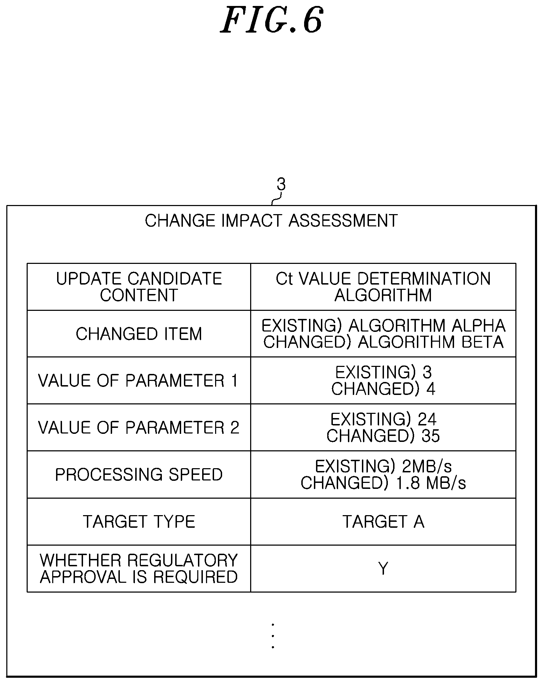

As illustrated in FIG. 6, the results of performing the change impact assessment 3 may include the update candidate content 2, the changed items, the values of the parameters, the processing speed, the target type, and whether the regulatory approval is required, etc.

For example, the results of performing the change impact assessment 3 may include information that the update candidate content 2 may be a 'Ct value determination algorithm', and the changed items may include information that 'algorithm alpha' included in the 'Ct value determination algorithm' is changed to 'algorithm beta'. The results of performing the change impact assessment 3 may include information that a value of parameter 1 is changed from the previous value of '3' to '4', a value of parameter 2 is changed from the previous value of '24' to '35', a processing speed is changed from the previous value of '2MB/s' to '1.8MB/s', the target type is information 'maintenance of target A', and whether the regulatory approval is required is the information 'Y'. The results of performing the change impact assessment 3 illustrated in FIG. 6 are one example and is not limited thereto.

Such change impact assessment 3 may be input to the research and development management device 100 for the purpose of assessing the change impact of the in vitro diagnostic reagent product.

The research and development management device 100 may control the research and development module 1 to be updated in response to the results of performing the change impact assessment 3. Responding to the results of the change impact assessment 3 for the update candidate content 2 refers to that the results of performing the change impact assessment satisfy a predetermined criterion.

In one embodiment, the predetermined criterion may be any one of the cases where the performance of the research and development module 1 according to the update candidate content 2 is the same as before, within a predetermined range, or outside the predetermined range, but is not limited thereto.

In one embodiment, the predetermined criterion may mean an update within a range where the update of the research and development module 1 does not affect the results of regulatory affair (RA) or quality assurance (QA).

In one embodiment, the predetermined criterion may specify that the result of the change impact assessment 3 is that the research and development module 1, which has been changed according to the update candidate content 2, has no change in the performance of the existing reagent product. For example, the predetermined criterion may be that the Ct value of the positive/negative determining result using the reagent product is not out of the predetermined range, the difference in the end relative fluorescence unit (RFU) value is not out of the predetermined range, the amplification data is formed in a sigmoid pattern, or the amplification efficiency is within the predetermined range.

In one embodiment, when a manufacturer of an extraction reagent is changed, the predetermined criterion may be that the performance of the extraction reagent after the change is assessed as equal to or better than the performance of the extraction reagent used previously.

In one embodiment, the predetermined criterion may be that the existing amplification performance is equivalent when the extraction equipment is changed or added, or when the equipment is changed or newly added.

In one embodiment, when a supplier of oligonucleotide is changed, in order to apply oligonucleotide of a new supplier, the predetermined criterion may be that the performance of the extraction reagent after the change is assessed as equal to or better than the performance of the extraction reagent used previously.

In one embodiment, when the end RFU of the change in the predetermined criterion is less than 5, and as the results of performing the change impact assessment 3 on the update candidate content 2, the changed RFU value is 4 RFU, the research and development management device 100 may control the research and development module 1 to update the research and development module 1 using the update candidate content 2.

In this case, when the research and development module 1 is directly operated in the research and development management device 100, the research and development management device 100 may control the research and development module 1 to be directly updated using the update candidate content 2.

Alternatively, when the research and development module 1 is distributed to and operated on the terminal of the external user account 200, the research and development management device 100 may control the research and development module 1 distributed to the external user account 200 to be updated using the update candidate content 2. For example, the research and development management device 100 may transmit a patch file or a control file, which may perform the update, to the terminal of the external user account 200 so that the research and development module 1 distributed to the external user account 200 is updated, or may distribute the research and development module 1, which has already been updated, to the terminal of the external user account 200.

According to one embodiment of the present disclosure, the external user account 200 participating in the reagent development through the research and development management device 100 of the in vitro diagnostic reagent product has an effect of using the gradually evolved research and development module. In addition, according to one embodiment, it is possible to obtain various data, which can evolve the research and development module 1, from the external user account 200.

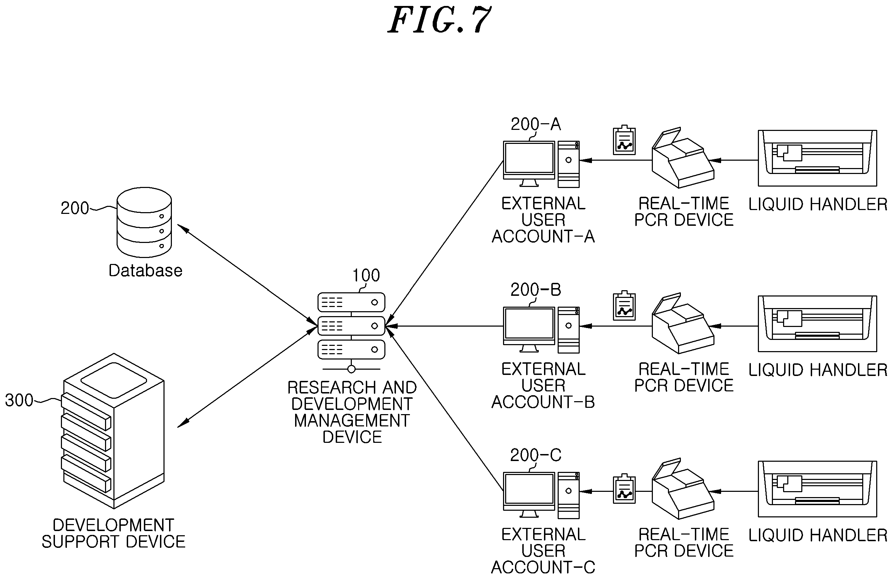

FIG. 7 is a diagram for describing the operation of the research and development management device 100 using the development support device that supports the operation of the research and development module 1 according to another embodiment.

As illustrated in FIG. 7, the research and development management device 100 may be further connected to the development support device via a network to obtain the research and development data and the clinical data from the development support device. The development support device may operate as a server that may communicate with the research and development management device 100, and may be composed of a smartphone, a desktop, a laptop, a tablet, a wearable device, etc., but is not limited thereto. In addition, contrary to what is illustrated, the development support device may be directly connected to the external user accounts 200 via the network.

In the development support device, the research and development module 1 may be operated. For example, the development support device may operate software that implements the algorithm for designing the oligonucleotide among the research and development modules 1 described above, the external user accounts 200 may access the development support device to perform the research and development, and the development support device may generate the research and development data and the clinical data. For example, the development support device may generate the target sequence data, experimental reaction results for the combination of the oligonucleotides, and the amplification data obtained from the nucleic acid amplification reaction.

The research and development management device 100 may obtain pieces of data generated by the development support device. As in the embodiment described above, the research and development management device 100 may determine the update candidate content 2 using the research and development data and the clinical data, and control the research and development module 1 to be updated in response to the results of performing the change impact assessment 3.

The structure and operation of the research and development management device of the in vitro diagnostic reagent product according to one embodiment have been described above.

Hereinafter, a method for updating a research and development module 1 of an in vitro diagnostic reagent product will be described. In one embodiment, the method for updating a research and development module 1 may be performed by a computing device. In this case, the computing device may be the research and development management device 100 described above. While describing the present disclosure according to one embodiment, operations similar to those performed by the research and development management device 100 described above will be omitted for description.

FIG. 10 is a diagram illustrating steps included in the method for updating a research and development module 1 according to one embodiment.

First, in step S 100, the research and development data of the in vitro diagnostic reagent product and/or the clinical data of the in vitro diagnostic reagent product, which is generated by the research and development module 1 used by the external user account 200, may be obtained.

Here, the external user account 200 may mean an account that uses the research and development equipment for research and development of the reagent product according to the same research and development process that allows the research and development of the reagent product to be performed using the same technology.

Here, the same technology may be at least one of a target sequence and amplicon determination technology, a candidate nucleotide design technology for primers and probes, an oligonucleotide structure, a signal generation mechanism indicating the presence of the target, a technology for differentiating multiple signals generated from one label in one channel, a signal processing technology, a positive/negative determination technology, or a nucleic acid extraction technology.

Here, the same research and development process may be a standardized research and development process provided by the research and development management device 100 of the in vitro diagnostic reagent product.

Here, the research and development equipment may be at least one of the nucleic acid extraction device, the liquid handler, and the real-time polymerase chain reaction (PCR) device.

The research and development equipment may be the same equipment, and the same equipment may be applied with the same calibration.

In one embodiment, the research and development module 1 may be software with the implemented algorithm for designing oligonucleotide used in the target nucleic acid amplification reaction. Alternatively, the research and development module 1 may be the software with the implemented algorithm used to process the amplification data obtained from the target nucleic acid amplification reaction. Alternatively, the research and development module 1 may be the software with the implemented algorithm used to determine the presence/absence of the target analytes in the sample. The research and development module 1 may be the software used to operate the research and development equipment. In this case, the research and development module 1 may be at least one of the nucleic acid extraction device, the liquid handler, and the real-time polymerase chain reaction (PCR) device.

The research and development module 1 may be provided in the research and development management device 100 so that the external user account 200 may access and use the research and development module 1, or may be software that is distributed from the research and development management device 100 to the terminal of the external user account 200.

In one embodiment, the research and development data and/or the clinical data of the in vitro diagnostic reagent product may be at least one of the target sequence data, the experimental reaction results for the combination of the oligonucleotides, the amplification data obtained from the nucleic acid amplification reaction, the trouble data generated during the research and development of the reagent product, the instructions executed to operate the research and development equipment and the values of parameters referenced in the execution of the instructions, or the trouble shooting data corresponding to the trouble data. Here, the driving of the research and development equipment may include sample preparation and nucleic acid extraction.

In step S200, the update candidate content 2 for the research and development module 1 determined at least partially based on the obtained research and development data and clinical data may be obtained.

The update candidate content 2 may include update content in which at least one selected from the group consisting of source codes, parameters, values of parameters, reference values for determining the presence or absence of the target analytes, and screen interfaces, which are included in the research and development module, is modified, generated, or deleted.

The update candidate content 2 may be the algorithm for designing the oligonucleotide used in the target nucleic acid amplification reaction included in the oligonucleotide design software. In one embodiment, the algorithm may be the algorithm for determining the region of the target sequence, the algorithm for designing the oligonucleotide, or the algorithm for calculating the assessment score for the performance of the oligonucleotides.

The update candidate content 2 may include at least one of the baselining algorithm, the amplification curve fitting algorithm, the Ct value determination algorithm, or the melting curve analysis algorithm.

The update candidate content 2 may include at least one of the algorithm for reading the presence/absence of the target analytes using the Ct value or the algorithm for determining the presence or absence of the target analytes using the fitting of amplification data obtained from the target nucleic acid amplification reaction.

In step S300, the results of performing the change impact assessment 3 of the update candidate content 2 on the research and development module 1 of the update candidate content 2 may be obtained.

In step S400, the research and development module 1 may be controlled to be updated in response to the results of performing the change impact assessment 3.

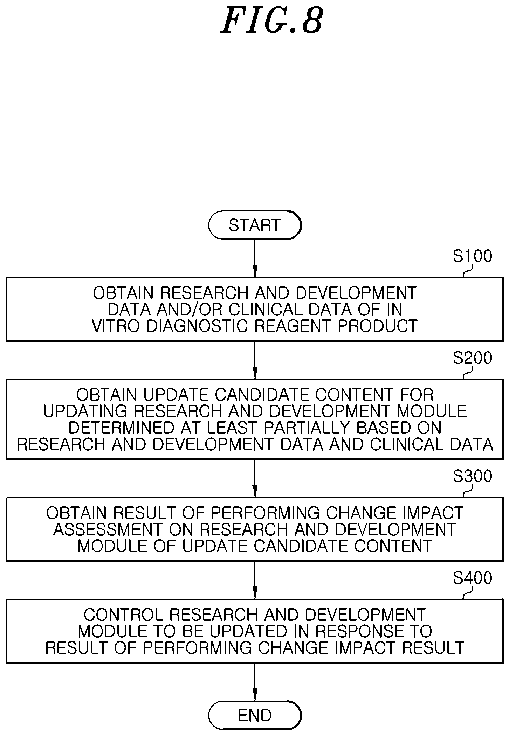

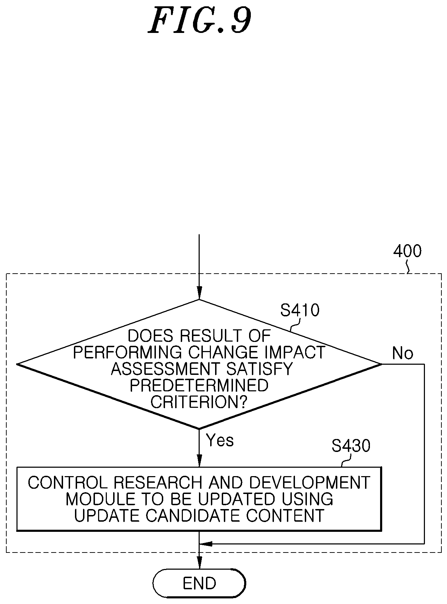

FIG. 9 is a flowchart for describing in more detail some steps of FIG. 8.

Referring to FIG. 9, when step S400 is performed, an operation of determining whether the results of performing the change impact assessment 3 in step S410 satisfy the predetermined criterion may be performed. When the results of performing the change impact assessment 3 in step S410 satisfy the predetermined criterion, the research and development module 1 may be controlled to be updated using the update candidate content 2 in step S430.

When the results of performing the change impact assessment 3 in step S410 do not satisfy the predetermined criterion, the research and development module 1 may be terminated without being updated.

The predetermined criterion may be any one of the cases where the performance of the research and development module 1 according to the update candidate content 2 is the same as before, within a predetermined range, or outside the predetermined range, but is not limited thereto.

In one embodiment, the predetermined criterion may refer to an update of the research and development module 1 that does not affect the results of the regulatory affair (RA) or the quality assurance (QA).

Meanwhile, the methods according to various embodiments described above may be implemented in the form of the computer program stored in the computer-readable recording medium programmed to perform each operation of the methods, and may also be implemented in the form of the computer-readable recording medium that stores the computer program programmed to perform each operation of the methods.

The above description is merely illustrative of the technical idea of the present disclosure, and those skilled in the art to which the present disclosure pertains will be able to make various modifications and variations without departing from the essential quality of the present disclosure. Accordingly, the embodiments disclosed in the present disclosure are not intended to limit the technical idea of the present disclosure, but to explain it, and the scope of the technical idea of the present disclosure is not limited by these embodiments. The protection scope of the present invention should be interpreted by the following claims, and all technical ideas within the scope equivalent thereto should be interpreted as being included in the scope of the present invention.

## Claims

1. A method for updating a research and development module of a research and development management device for developing an in vitro diagnostic reagent product, the method comprising:
obtaining by the research and development management device, research and development data and/or clinical data of the in vitro diagnostic reagent product generated by the research and development module used by an external user account;
obtaining update candidate content for updating the research and development module determined at least partially based on the obtained research and development data and clinical data, wherein the update candidate content includes update content in which at least one selected from the group consisting of a source code, a parameter, a value of a parameter, a reference value for determining presence or absence of a target analyte, and a screen interface, which are included in the research and development module, is modified, created, or deleted;
obtaining a result of performing change impact assessment of the update candidate content on the research and development module; and
controlling the research and development module to be updated in response to the result of performing the change impact assessment.

2. The method for updating the research and development module of claim 1, wherein the change impact assessment for the research and development module is an impact assessment for performance of the in vitro diagnostic reagent product using the research and development module.

3. The method for updating the research and development module of claim 2, wherein the in vitro diagnostic reagent product is a multiplex molecular diagnostic reagent product for detecting a plurality of targets,
the update candidate content is update content for improving detection of at least one of the plurality of targets, and
the change impact assessment for the research and development module is to assess whether detection results for other targets than the at least one target with improved detection results are not changed in detection using the research and development module to which the update candidate content is applied.

4. The method for updating the research and development module of claim 1, wherein the in vitro diagnostic reagent product is a molecular diagnostic reagent product, and
the change impact assessment is performed using at least one selected from the group consisting of a Ct value, an End-RFU value, sensitivity, and a positive/negative determination result from use in the molecular diagnostic reagent product.

5. The method for updating the research and development module of claim 1, wherein the in vitro diagnostic reagent product is a molecular diagnostic reagent product, and
the research and development module includes software with an implemented algorithm for designing an oligonucleotide used in a target nucleic acid amplification reaction.

6. The method for updating the research and development module of claim 5, wherein the update candidate content is for the algorithm for designing the oligonucleotide, and
the algorithm for designing the oligonucleotide includes at least one selected from the group consisting of an algorithm for collecting and aligning a target sequence, an algorithm for determining an oligonucleotide designable region of the target sequence and an algorithm for calculating an assessment score for performance of the oligonucleotide.

7. The method for updating the research and development module of claim 1, wherein the in vitro diagnostic reagent product is a molecular diagnostic reagent product, and
the research and development module includes software with an implemented algorithm used to process and analyzing amplification data obtained from a target nucleic acid amplification reaction.

8. The method for updating the research and development module of claim 7, wherein the update candidate content includes at least one selected from the group consisting of a baselining algorithm, an amplification curve fitting algorithm, a melting curve analysis algorithm, an algorithm for determining presence or absence of a target analyte using a Ct value and an algorithm for determining the presence or absence of the target analyte using an amplification curve fitting result.

9. The method for updating the research and development module of claim 1, wherein the research and development module includes software for displaying an amplification curve representing an amplification reaction result of amplification data obtained from a target nucleic acid amplification reaction and a result of determining presence or absence of the target, and
the update candidate content includes a screen interface component for displaying the amplification curve and the result of determining the presence or absence of the target.

10. The method for updating the research and development module of claim 1, wherein the research and development module includes software to operate research and development equipment.

11. The method for updating the research and development module of claim 10, wherein the operation of the research and development equipment is executed for sample preparation and nucleic acid extraction.

12. The method for updating the research and development module of claim 1, wherein the external user account is an account that uses research and development equipment for research and development of the reagent product according to the same research and development process so that the research and development of the reagent product is performed using the same technology.

13. The method for updating the research and development module of claim 12, wherein the same technology includes at least one technology selected from the group consisting of the following technologies:
a technology for determining a target sequence and an oligonucleotide designable region; an oligonucleotide candidates design technology for a primer and a probe; an oligonucleotide structure technology; a signal generation mechanism indicating presence of a target; a technology for differentiating multiple signals generated from one label in one channel; a signal processing technology; a positive/negative determination technology; and a nucleic acid extraction technology.

14. The method for updating the research and development module of claim 12, wherein a same research and development process is a standardized research and development process provided by the research and development management device of the in vitro diagnostic reagent product.

15. The method for updating the research and development module of claim 12, wherein the research and development equipment includes at least one selected from the group consisting of a nucleic acid extraction device, a liquid handler, and a real-time polymerase chain reaction (PCR) device.

16. The method for updating the research and development module of claim 15, wherein the research and development equipment is the same equipment, and the same equipment is calibrated with the same calibration.

17. The method for updating the research and development module of claim 1, wherein the research and development module is provided in the research and development management device so that the external user account accesses and uses the research and development module, or is distributed from the research and development management device to a terminal of the external user account.

18. The method for updating the research and development module of claim 1, wherein the in vitro diagnostic reagent product is a molecular diagnostic reagent product, and
the research and development data and/or the clinical data of the in vitro diagnostic reagent product includes at least one selected form the group consisting of target sequence data, a performance test result for oligonucleotide, amplification data obtained from a nucleic acid amplification reaction, trouble data generated during research and development of the reagent product, an instruction executed to operate research and development equipment, a value of a parameter referenced in the execution of the instructions, and trouble shooting data corresponding to the trouble data.

19. The method for updating the research and development module of claim 1, wherein the research and development data and/or the clinical data of the in vitro diagnostic reagent product is obtained from a research and development support module included in the research and development management device and/or a research and development support device connected to the research and development management device via a communication network.

20. The method for updating the research and development module of claim 1, wherein when the result of performing the change impact assessment satisfies a predetermined criterion, the controlling of the research and development module to be updated is performed to updeate the research and development module.

21. A research and development management device, comprising:
a memory that stores at least one instruction; and
a processor,
wherein, by executing the at least one instruction, the processor controls a research and development management device to obtain research and development data of an in vitro diagnostic reagent product and/or clinical data of the in vitro diagnostic reagent product that is generated by a research and development module used by an external user account,
obtain update candidate content for updating the research and development module determined at least partially based on the obtained research and development data and clinical data, the update candidate content including update content in which at least one selected from the group consisting of a source code, a parameter, a value of a parameter, a reference value for determining presence or absence of a target analyte, and a screen interface, which are included in the research and development module, is modified, created, or deleted,
obtain a result of performing change impact assessment of the update candidate content on the research and development module, and
update the research and development module in response to the result of performing the change impact assessment.

22. A computer-readable recording medium storing a computer program,
wherein the computer program is programmed to perform the following steps:
obtaining, by a research and development management device, research and development data and/or clinical data of the in vitro diagnostic reagent product generated by a research and development module used by an external user account;
obtaining update candidate content for updating the research and development module determined at least partially based on the obtained research and development data and clinical data, wiherein the update candidate content includes update content in which at least one selected from the group consisting of a source code, a parameter, a value of a parameter, a reference value for determining presence or absence of a target analyte, and a screen interface, which are included in the research and development module, is modified, created, or deleted;
obtaining a result of performing change impact assessment of the update candidate content on the research and development module; and
controlling the research and development module to be updated in response to the result of performing the change impact assessment.

23. A computer program stored in a computer-readable recording medium, wherein
the computer program is programmed to perform the following steps:
obtaining, by a research and development management device, research and development data and/or clinical data of the in vitro diagnostic reagent product generated by a research and development module used by an external user account,
obtaining update candidate content for updating the research and development module determined at least partially based on the obtained research and development data and clinical data, wherein the update candidate content includes update content in which at least one selected from the group consisting of a source code, a parameter, a value of a parameter, a reference value for determining presence or absence of a target analyte, and a screen interface, which are included in the research and development module, is modified, created, or deleted;
obtaining a result of performing change impact assessment of the update candidate content on the research and development module; and
controlling the research and development module to be updated in response to the result of performing the change impact assessment.
